# EUROPEAN PATENT APPLICATION

(11) **EP 4 112 114 A1**
(43) Date of publication of application: **04.01.2023**
(21) Application number: 22156436.2
(22) Date of filing: 11.02.2022
(51) Int. Cl.: A61M 39/10, A61J 15/00, A61M 39/12

(54) **CONNECTOR FOR A GASTROSTOMY DEVICE**

(30) Priority: 02.07.2021 GB 202109572
(71) Applicant: GBUK Group Limited, Selby, North Yorkshire YO8 5DD (GB)
(72) Inventor: COWAN, Joseph, Selby, YO8 5DD (GB); ALLSOPP, Ross, Selby, YO8 5DD (GB)
(74) Representative: Neilson, Martin Mark

(57) **Abstract**

A connector (1) for coupling an enteral feeding solution supply tube to a port of a gastrostomy device, comprising a fluid conduit (10) having an upstream end (33) and a downstream end; and a nozzle (2) housing a first portion of the fluid conduit at the downstream end configured to be connectable to an orifice defined in the port of the gastrostomy device. The nozzle has a proximal end (21), a distal end (20) and a longitudinal axis extending therebetween. An external diameter of the nozzle at or adjacent the distal end is substantially equal to an external diameter of the nozzle at or adjacent the proximal end. An external surface of the nozzle includes one or more indentations (24). The invention also relates to a feeding extension set including a connector and an enteral feeding solution supply tube.

## Description

### Field of invention

The present invention relates to a connector for connecting a supply tube for an enteral feeding solution to a gastrostomy device.

### Background art

Patients with chronic conditions such as certain neurologic and neuromuscular disorders, malformations of the mouth and oesophagus, as well as critically ill patients in intensive care units or patients recovering from surgery are often unable to suck or swallow and may therefore be unable to take adequate nutrients or medicines orally. In these situations a gastrostomy device is implanted in a patient's stomach wall to provide nutrition (and in some cases medication) directly into the stomach.

The gastrostomy device can be a long tube (*e.g.* a PEG tube) which includes a tube portion which extends outside the patient's body. These types of tubes are typically used initially or over shorter time periods. For longer term enteral feeding, low profile (*e.g.* "button") tubes are often used. These have a low profile "button" connector which lies fairly flush against the patient's body. A feeding tube can then be attached to the button connector to administer nutrition or medication when required. The lack of a permanent external tube makes button tubes more attractive for long-term enteral feeding as the risk of snagging is reduced, and the button is less visible and can more easily be concealed under clothing. Balloon buttons (which are held in place by a fluid-filled balloon) can also be replaced at home after training, without medical assistance.

Feeding tubes typically have a male connector on the end which mates with a female port on the button connector. Existing male connectors such as the connector disclosed in GB2586690A typically include a tapered nozzle end which is pushed into the button connector to open a non-return valve (such as a duckbill valve) and allow feed to pass through. The tapered nozzle end reduces friction and wear on elastomeric parts (such as the non-return valve and sealing ring) during insertion and removal of the device. However, the tapered male connector could mate with or be pushed into a female connector of the incorrect (*i.e.* smaller) size. A patient may have multiple connectors of different sizes, each of which performs a different function (such as provision of medication, fluid draining *etc*)*.* A misconnection could lead to feed or medication being provided through the wrong portal (such as an IV line), with potentially lethal results. Such misconnections are referred to in medical treatment as *"never events"* - a serious event which should never happen and is largely preventable.

### Summary of the Invention

The present invention seeks to provide a connector for enteral feeding which serves to couple reliably an enteral feeding solution supply tube to a port of a gastrostomy device. In particular, the connector seeks (for example) to avoid inadvertent misconnection and not appreciably increase wear and tear on internal components of the female button connector.

Viewed from a first aspect the present invention provides a connector for coupling an enteral feeding solution supply tube to a port of a gastrostomy device, wherein the connector comprises: a fluid conduit having an upstream end and a downstream end; a nozzle housing a first portion of the fluid conduit at the downstream end configured to be connectable to an orifice defined in the port of the gastrostomy device, wherein the nozzle has a proximal end, a distal end and a longitudinal axis extending therebetween, wherein an external diameter of the nozzle at or adjacent the distal end is substantially equal to an external diameter of the nozzle at or adjacent the proximal end, and wherein an external surface of the nozzle includes one or more indentations.

In use, the nozzle may be inserted into a respective orifice on the gastrostomy device. The nozzle may be configured to fit snugly (*e.g.* in a tight friction fit) within the orifice, *i.e.* the external dimensions of the nozzle may be slightly smaller than the internal dimensions of the orifice. The orifice may be provided with a sealing ring and a non-return valve. The dimensions of the nozzle may be such that when inserted into the orifice, the sealing ring contacts the external surface towards the proximal end and provides a fluid seal. The nozzle also may have a length sufficient for the distal end of the nozzle to push against and fully open the non-return valve when inserted to enable fluid flow. When the nozzle is removed from the orifice, the non-return valve is closed and backflow is prevented.

As the nozzle is pushed into or pulled out of the orifice, the sealing ring may be dragged along the length of the external surface between the distal and proximal ends (during insertion) or vice versa (during removal). Over time and repeated connection/disconnection this contact will cause wear to the sealing ring. The indentations according to the connector of the invention advantageously reduce the surface area of the nozzle which is in contact with the sealing ring and other elastomeric components of the port on the gastrostomy device, thus prolonging the lifetime of these components.

Preferably the nozzle and the respective orifice on the gastrostomy device have a circular cross section. A circular cross-section is advantageous as the connector then need not have a fixed orientation and can rotate within the orifice as required.

The indentations may extend longitudinally for a first distance from a first point at or towards the distal end to a second point between the distal end and the proximal end.

The second point is preferably longitudinally spaced apart from the proximal end by a second distance to define a contact area on the external surface of the nozzle which is substantially free from indentations. This ensures that the sealing ring contacts and provides a fluid seal around the entire circumference of the nozzle when the connector is connected to the port of the gastrostomy device.

Preferably there are multiple indentations spaced apart circumferentially around the external surface and circumferentially separated by ridges (*i.e.* regions of the external surface which are not indented and which define the outer diameter of the nozzle). The circumferential extent of each indentation may be defined by its arc on an external circumference of the nozzle. There may be any number of indentations, each having the same or a different arc. Preferably the sum of the circumferential extent of each arc is between 50% and 90% of the external circumference of the nozzle. Preferably there are between 2 and four indentations having an equal circumferential extent (arc). Even more preferably there are four equally spaced indentations separated by a total of four ridges, wherein each indentation has a circumferential extent of between 15% and 20% of the external circumference.

The circumferential extent of each ridge may be defined by its arc on an external surface of the nozzle. Preferably the sum of the circumferential extent (arc) of each ridge is between 10% and 50% of the external circumference of the nozzle. Even more preferably the sum of the arc of each ridge is between 20% and 40% of the external circumference of the nozzle. Preferably the arc of each individual ridge is not less than 5% of the external circumference of the nozzle. This ensures that the ridges have sufficient structural rigidity to not deform if a user attempts to force the nozzle into an orifice which is too small.

The first point is preferably at the distal end, such that the indentations extend from the distal end of the nozzle towards the proximal end.

Preferably a first longitudinal distance between the distal end and the second point is greater than a second longitudinal distance between the second point and the proximal end (*i.e.* the second point is preferably closer to the proximal end than the distal end). Preferably the first longitudinal distance is 1.5 to 3 times longer than the second longitudinal distance. Even more preferably the first longitudinal distance is about 2 times longer than the second longitudinal distance.

The nozzle may have a rounded edge at the distal end to aid insertion into the orifice of the gastrostomy device. Where the distal end has a rounded edge, the rounded edge preferably has a longitudinal extent of less than 5% the length of the nozzle.

The connector may further comprise a body housing a second portion of the fluid conduit at the upstream end, wherein the upstream end of the fluid conduit is configured for connection to an enteral feeding solution supply tube.

The nozzle may have a fillet at the proximal end to increase the structural rigidity of the connection between the nozzle and the body. The fillet preferably has a longitudinal extent of less than 5% of the length of the nozzle.

The external diameter of the nozzle at or adjacent the distal end (*i.e.* the diameter measured at the distal end where there is no rounded edge, or the diameter of the nozzle adjacent any rounded edge) is substantially the same as the external diameter of the nozzle at or adjacent the proximal end (*i.e.* the diameter measured at the proximal end where there is no fillet, or the diameter of the nozzle adjacent any fillet). In other words, the nozzle does not have a significant taper between the proximal end and distal end owing to regions such as the contact area and ridges between the indentations which together define the external diameter of the nozzle along its length.

This advantageously means that the nozzle cannot (as it does not have a smaller diameter at the distal end) be inserted or partially inserted (deliberately or accidentally) into an orifice which is smaller than that for which it is designed. The ridges prevent the nozzle being forced into a smaller orifice. This helps to reduce the risk of misconnection errors and "never events".

Preferably the nozzle has a substantially constant external diameter along its entire length between the proximal and distal ends, excluding any rounded edge or fillet where present.

Preferably an internal diameter of the nozzle (*i.e.* the diameter of the fluid conduit in the nozzle) is substantially constant along the entire length between the proximal and distal ends.

The second portion of the fluid conduit may be substantially coaxial with the first portion of the fluid conduit. Alternatively the second portion of the fluid conduit may be substantially perpendicular to the first portion of the fluid conduit. This advantageously reduces the risk of the connector being prematurely disconnected if the enteral feeding solution supply pipe is snagged or pulled. The second portion of the fluid conduit may have the same internal diameter as the first portion, or may have a larger or smaller internal diameter. Preferably the second portion has the same or a larger internal diameter than the first portion.

The connector may further comprise an attachment mechanism provided at or adjacent the proximal end of the nozzle to releasably engage with a corresponding element on the gastrostomy device to secure the connector in place during feeding. The attachment mechanism may be provided on the body. The attachment mechanism may include a pair of arms circumferentially arranged around the proximal end of the nozzle, each arm including a first free end and a second free end, wherein the second free end includes a catch configured to releasably engage with a circumferential rim on the port of the gastrostomy device. Each arm may be connected to the body by a flexible bridge about which the first and second free ends of each arm can be pivoted.

The catch may be a ledge located on the inner wall of the arm that projects inwardly towards the nozzle. The ledge may have a rim-contacting surface configured to releasably engage with a ledge-contacting surface of the circumferential rim.

The connector may further comprise a lock member, at least a part of which is configured to be sandwiched between the body and/or proximal end and an inner wall of each arm. The lock member may be removably attached to the connector.

The connector may be moulded as a unitary component from a plastic material such as polyoxymethylene, polyurethane, high density polyethylene, polyvinyl chloride or polypropy lene.

Viewed from a second aspect the present invention provides a feeding extension set, comprising: an enteral feeding solution supply tube; and a connector as hereinbefore defined, wherein the connector is configured for coupling the enteral feeding solution supply tube to a port of a gastrostomy device.

Viewed from a third aspect the present invention provides a method of delivering an enteral fluid to a patient comprising the following steps:
(i) taking a connector as hereinbefore defined;
(ii) inserting the nozzle of the connector into a corresponding port on an indwelling gastrostomy device;
(iii) coupling the upstream end of the fluid conduit to an enteral feeding solution supply tube;
(iv) providing an enteral feeding solution to the patient's stomach through the feeding solution supply tube to and through the connector and gastrostomy device.

### Brief description of drawings

A specific implementation of the present invention will now be described, by way of example only, and with reference to the accompanying drawings in which:
**Figure 1** is a side view of a connector according to a first embodiment.
**Figure 2** is an enlarged view of part of Figure 1 showing the nozzle of the connector.
**Figure 3** is a perspective view of the connector of Figure 1.
**Figure 4** is an underside view of the connector of Figure 1.
**Figure 5** is a cross-section of part of the connector of Figure 1 partially inserted into a port on a gastrostomy device.
**Figure 6** is a side view of a connector according to a second embodiment.
**Figure 7** is a perspective view of the connector of Figure 6.
**Figure 8** is a cross-section of part of the connector of Figure 6 partially inserted into a port on a gastrostomy device.

### Detailed description of a preferred embodiment of the invention

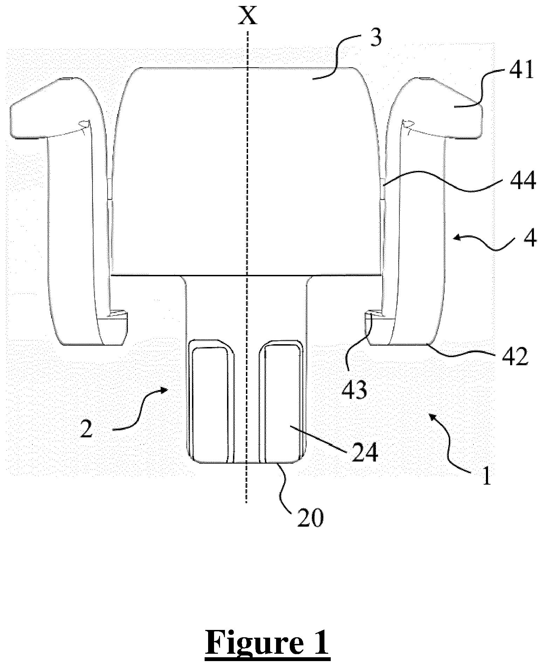

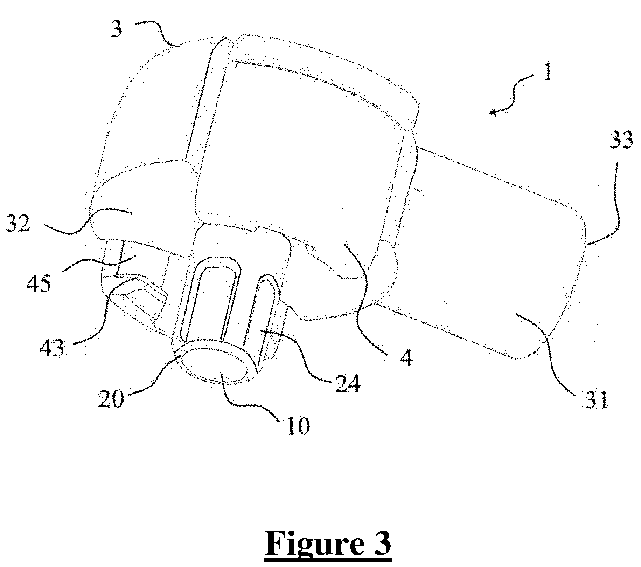

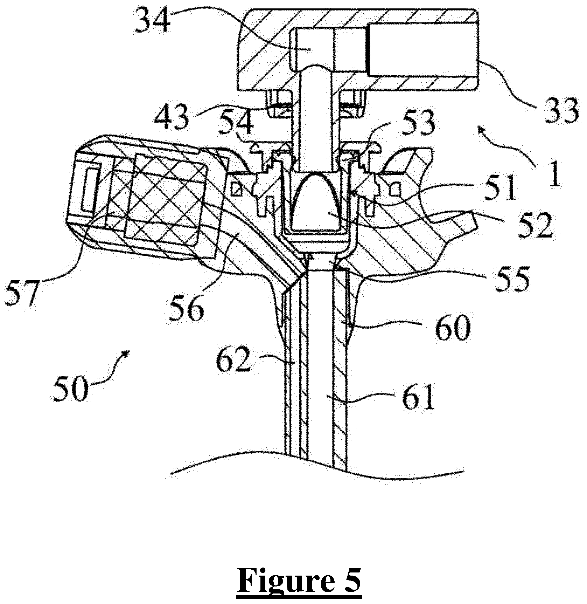

Referring initially to Figures 1 and 3 a connector 1 for coupling an enteral feeding solution supply tube to a port of a gastrostomy device is shown. The connector 1 includes a nozzle 2 which extends from an underside face 32 of a body 3 and is substantially cylindrical with a circular cross-section. A fluid conduit 10 extends through the nozzle 2 and body 3 from a distal end 20 of the nozzle 2 to an upstream end 33 of a shank portion 31 of the body 3. In this particular embodiment the shank portion 31 extends along an axis which is substantially perpendicular to a longitudinal axis X of the nozzle 2. The fluid conduit 10 therefore includes a substantially L-shaped junction 34 (shown in Figure 5) within the body 3.

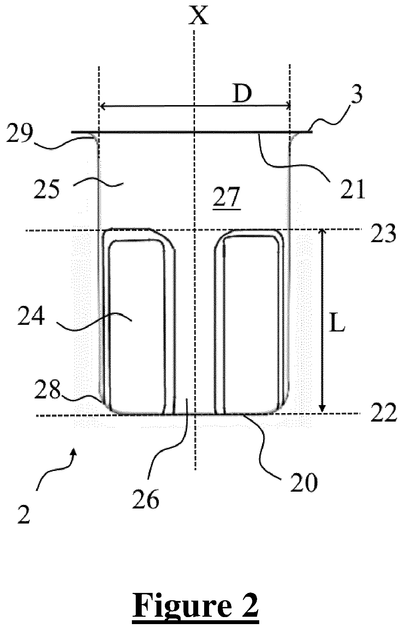

With reference now to Figure 2, the nozzle 2 extends along the longitudinal axis X between the distal end 20 and a proximal end 21 adjacent the body 3. The nozzle 2 has a diameter D which is constant along substantially the entire distance between the distal end 20 and the proximal end 21, with the exception of a rounded edge 28 at the distal end 20 which has a longitudinal extent of less than 5% of the distance between the distal end 20 and proximal end 21. A fillet 29 is also provided at the proximal end 21 which has a longitudinal extent less than 5% of the distance between the distal end 20 and the proximal end 21. The fillet 29 is provided to improve the structural rigidity of the connector 1. The nozzle 2 therefore has a diameter D which is constant along greater than 90% of its length.

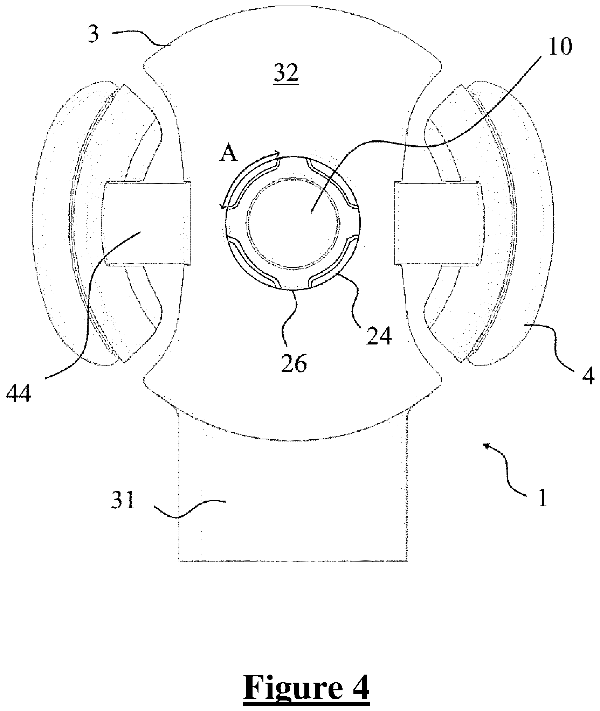

The nozzle 2 has an exterior surface 27. As best shown in Figure 4, four indentations 24 are spaced circumferentially around the exterior surface 27. Each indentation 24 extends circumferentially by a distance A which is an arc on the circumference of the nozzle 2. As best shown in Figure 2, each indentation 24 extends longitudinally for a distance L between a first point 22 and a second point 23. In this particular embodiment, the first point 22 is at the distal end 20 of the nozzle 2 (*i.e.* the indentations 24 extend to the distal end 20), and each of the four indentations 24 have the same longitudinal extent (L) and circumferential extent (A). The four indentations 24 are equally spaced circumferentially, with ridges 26 between the indentations 24. Around the circumference of the nozzle 2, between the first point 22 and the second point 23 the indentations 24 account for approximately 70% of the area of the exterior surface 27, with the ridges 26 accounting for the remainder of the area of the exterior surface 27.

In this embodiment, the longitudinal extent (L) is equivalent to approximately two-thirds of the distance between the distal end 20 and the proximal end 21.

Between the second point 23 and the proximal end 21, the exterior surface 27 is substantially free from indentations. This defines a contact region 25.

With reference now to Figures 1 and 4, the connector 1 includes an attachment mechanism in the form of a pair of arms 4 provided on the body 3. Each arm 4 is attached to the body 3 by a flexible bridge 44 about which the arm 4 can pivot. Each arm 4 has a first free end 41 and a second free end 42. As best shown in Figure 1, the second free end 42 of each arm 4 is spaced apart from and annularly extends partially around the contact region 25 of the nozzle 2. The second free end 42 includes a catch 43 on an inwards facing wall 45 of the arm. The catch 43 is a ledge that projects inwardly towards the nozzle 2.

The connector 1 is moulded as a unitary component from polyurethane.

With reference now to Figure 5, the connector 1 can be connected to a port 51 on a gastrostomy device 50. The gastrostomy device 50 has a low-profile "button" connector and is a balloon gastrostomy device which includes a balloon inflation port 57 which connects to a secondary channel 62 of a feeding tube 60 via a conduit 56. The feeding tube 60 includes a main channel 61 for the supply of feed. The main channel 61 is in fluid communication with a void 55 downstream of a non-return valve 52 in the port 51.

The non-return valve 52 in this embodiment is a duckbill valve. A sealing ring 53 extends annularly around the entrance to the port 51. The sealing ring 53 and duckbill valve are composed of an elastomeric material. An outwardly facing rim 54 composed of a rigid plastic material extends circumferentially around the port 51.

The connector 1 is connected to the gastrostomy device 50 by inserting the nozzle 2 into the port 51. As the nozzle 2 is inserted, the distal end 20 contacts and pushes against the duckbill valve 52. The catches 43 on the arms 4 also contact the circumferential rim 54. As shown in Figure 5, both the catches 43 and the rim 54 have rounded edges. As these components are pushed together, the second free end 42 of each arm 4 is forced outwards by the rounded edges contacting and sliding against each other. The arms 4 pivot about the flexible bridge 44, with the first free end 41 moving inwardly towards the body 3.

As the connector 1 is pushed further downwards from the position shown in Figure 5, the outward movement of the second free end 42 allows the catch 43 to slide past and underneath the rim 54. The rim 54 then prevents upwards movement of the connector 54, thereby locking the connector 1 onto the port 51 in a locked position to prevent premature disconnection. To release the connector 1, a user squeezes the first free end 41 of each arm 4 to release the catch 43, and then pulls the connector 1 away from the gastrostomy device 50.

During insertion of the nozzle 2 into the port 51, the distal end 20 pushes against and opens the duckbill valve 52. The duckbill valve 52 is positioned within the port 51 to prevent accidental opening or damage. The nozzle 2 is long enough to penetrate and fully open the duckbill valve 52.

Once the connector 1 is locked in position on the gastrostomy device 50 and the duckbill valve 52 is open, feed can be supplied via an enteral feeding solution supply tube attached to the upstream end 33 at the shank portion 31 through the fluid conduit 10, the duckbill valve 52, void 55 and into the main channel 61 of the feeding tube 60.

When in the locked position, the sealing ring 53 is in a tight friction fit with the contact region 25, creating a fluid seal to prevent fluid leaking from the port 51. During insertion and removal, the sealing ring 53 is dragged along the exterior surface 27 of the nozzle 2. The indentations 25 (not visible in Figure 5) reduce the surface area in contact with the sealing ring 53 during insertion and removal. This reduces the amount of force required to insert and remove the connector 1 and also reduces wear on the sealing ring 53, thus prolonging the life of the gastrostomy device 50.

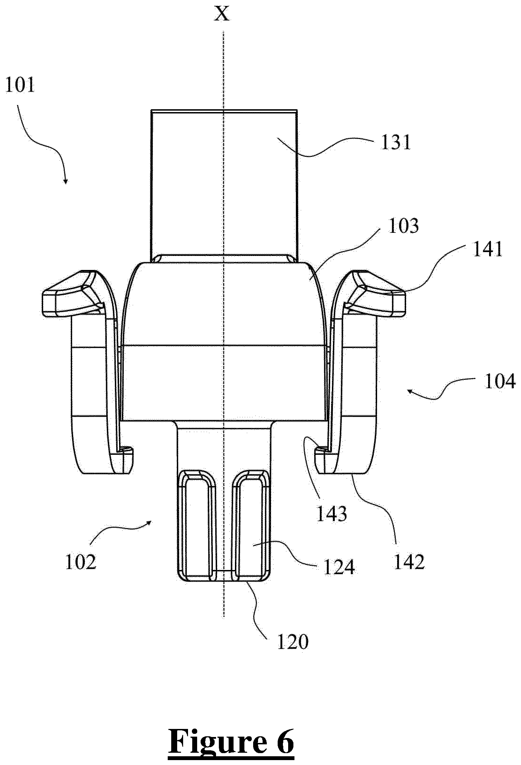

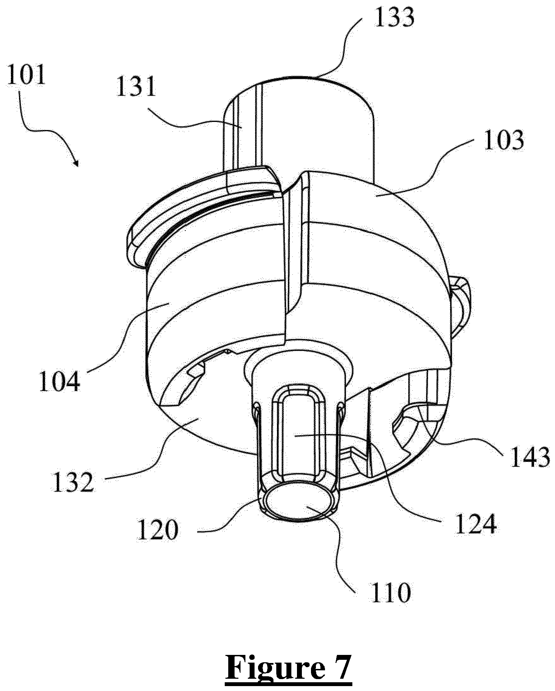

Referring now to Figures 6 and 7, an alternative connector 101 is shown. The connector 101 has the same nozzle 102, body 103 and arm 104 features as the connector 1. However in the connector 101, the fluid conduit 110 extends through the nozzle 102 and body 103 from a distal end 120 of the nozzle 102 to an upstream end 133 of a shank portion 131 of the body 3, and the shank portion 31 extends substantially coaxially with a longitudinal axis X of the nozzle 2. The fluid conduit 110 is therefore substantially straight between the upstream end 133 and the distal end of the nozzle 102. This configuration may be preferable for certain patients depending on their circumstances.

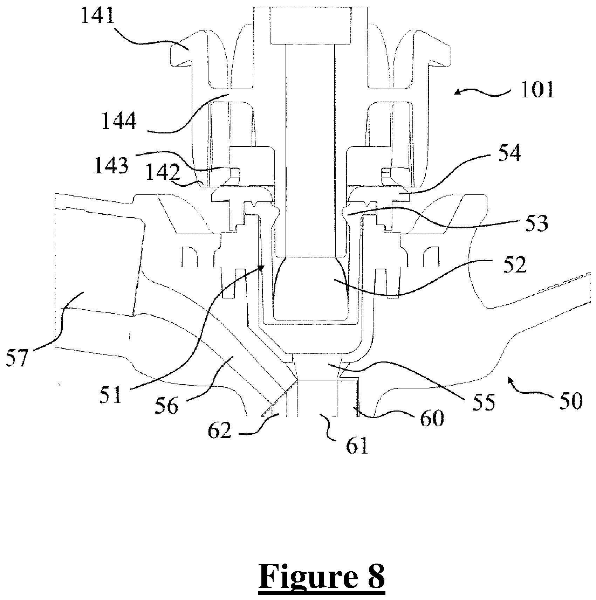

As shown in Figure 8, the connector 101 is attachable to the gastrostomy device 50 in the same manner as the connector 1. The fluid conduit 110 is aligned with the main channel 161 of the feeding tube 160. In this particular embodiment the inner diameter of the fluid conduit at the upstream end 133 is larger than the internal diameter at the downstream end.

## Claims

1. A connector for coupling an enteral feeding solution supply tube to a port of a gastrostomy device, wherein the connector comprises:
a fluid conduit having an upstream end and a downstream end;
a nozzle housing a first portion of the fluid conduit at the downstream end configured to be connectable to an orifice defined in the port of the gastrostomy device, wherein the nozzle has a proximal end, a distal end and a longitudinal axis extending therebetween,
wherein an external diameter of the nozzle at or adjacent the distal end is substantially equal to an external diameter of the nozzle at or adjacent the proximal end, and wherein an external surface of the nozzle includes one or more indentations.

2. The connector of claim 1, wherein the nozzle has a substantially circular cross section.

3. The connector of claim 1 or 2, wherein the external surface of the nozzle includes multiple circumferentially spaced apart indentations.

4. The connector of claim 3, wherein the circumferential extent of each indentation is defined by its arc on the external circumference of the nozzle, and wherein the sum of the circumferential extent (arc) of each indentation gives a total circumferential extent of between 50% and 90% of the external circumference of the nozzle.

5. The connector of claim 3, wherein the circumferential extent of each indentation is defined by its arc on the external circumference of the nozzle, and wherein each indentation has a circumferential extent (arc) of between 15% and 20% of the external circumference of the nozzle.

6. The connector of any preceding claim, wherein each indentation extends longitudinally between a first point at the distal end and a second point between the distal end and the proximal end.

7. The connector of claim 6, wherein a first longitudinal distance between the distal end and the second point is greater than a second longitudinal distance between the second point and the proximal end.

8. The connector of claim 7, wherein the first longitudinal distance is between 1.5 and 3 times longer than the second longitudinal distance.

9. The connector of any preceding claim, further comprising a body housing a second portion of the fluid conduit at the upstream end, wherein the upstream end is configured to be connectable to the enteral feeding solution supply tube.

10. The connector of claim 9, wherein the second portion of the fluid conduit is substantially perpendicular to the first portion of the fluid conduit.

11. The connector of claim 9, wherein the second portion of the fluid conduit is substantially coaxial with the first portion of the fluid conduit.

12. The connector of any preceding claim, further comprising an attachment mechanism provided at or adjacent the proximal end of the nozzle to releasably engage with a corresponding element on the gastrostomy device to secure the connector in place during feeding.

13. The connector of claim 12 wherein the attachment mechanism comprises a pair of arms circumferentially arranged around the proximal end of the nozzle, wherein each arm includes a first free end and a second free end, wherein the second free end includes a catch configured to releasably engage with a circumferential rim on the port of the gastrostomy device, and wherein each arm is connected to the body by a flexible bridge about which the first and second free ends can be pivoted.

14. A feeding extension set comprising:
an enteral feeding solution supply tube; and
a connector according to any of claims 1 to 12, wherein the connector is configured for coupling the enteral feeding solution supply tube to a port of a gastrostomy device.

15. A method of delivering an enteral fluid to a patient comprising:
(i) taking a connector according to claims 1 to 12;
(ii) inserting the nozzle of the connector into a corresponding port on an indwelling gastrostomy device;
(iii) coupling the upstream end of the fluid conduit to an enteral feeding solution supply tube; and
(iv) providing an enteral feeding solution to the patient's stomach through the feeding solution supply tube to and through the connector and gastrostomy device.
